# EUROPEAN PATENT SPECIFICATION

(11) **EP 1 906 931 B1**
(45) Date of publication and mention of the grant of the patent: **03.12.2008**
(21) Application number: 06765392.3
(22) Date of filing: 29.06.2006
(51) Int. Cl.: A61K 9/20, A61K 47/34, A61K 31/401

(54) **IMPROVED PHARMACEUTICAL COMPOSITION CONTAINING ACE INHIBITOR AND METHOD FOR THE PREPARATION THEREOF**
VERBESSERTE PHARMAZEUTISCHE ZUSAMMENSETZUNG MIT EINEM ACE-HEMMER UND HERSTELLUNGSVERFAHREN DAFÜR
COMPOSITION PHARMACEUTIQUE AMELIOREE CONTENANT UN INHIBITEUR ACE ET PROCEDE DE PREPARATION DE LADITE COMPOSITION

(30) Priority: 30.06.2005 GR 20050100332
(43) Date of publication of application: 09.04.2008
(73) Proprietor: Pharmathen S.A., Pallini, Attikis 15351 (GR)
(72) Inventor: KARAVAS, Evangelos, GR-153 51 Pallini Attikis (GR); KOUTRIS, Efthimios, GR-153 51 Pallini Attikis (GR); KOTZAGIORGIS, Evangelos, GR-653 02 Kavala (GR); BIKIARIS, Dimitrios, Gr-543 51 Thessaloniki (GR)
(86) International application number: PCT/GR2006/000033
(87) International publication number: WO 2007/003972

(56) References cited:
- EP-A- 0 264 888
- EP-B- 0 280 999
- EP-B- 0 408 273
- WO-A-00/44353
- NG F H ET AL: "Effect of simethicone on the accuracy of the rapid urease test." EUROPEAN JOURNAL OF GASTROENTEROLOGY & HEPATOLOGY. OCT 1998, vol. 10, no. 10, October 1998 (1998-10), pages 851-854, XP009062928 ISSN: 0954-691X

## Description

### TECHNICAL FIELD OF THE INVENTION

The present invention relates to improved dosage forms such as tablets and capsules and in particular to a formulation for oral or sub-lingual administration comprising a therapeutically effective quantity of an ACE inhibitor, and more particularly Fosinopril, Trandolapril or salts thereof, in combination with inorganic silica polymer such as Dimethicone and the preparation thereof.

### BACKGROUND OF THE INVENTION

Angiotensin Converting Enzyme (ACE) inhibitors are well known in the art for their activity for inhibiting angiotensin converting enzyme, thereby blocking conversion of the angiotensin I to angiotensin II. The principal pharmacological and clinical effects of ACE inhibitors arise from suppression of synthesis of angiotensin II. Angiotensin II is a potent pressor substance and, therefore, blood pressure lowering can result from inhibition of this biosynthesis, especially in cases wherein hypertension is angiotensin II related. ACE inhibitors are effective antihypertensive agents and clinically useful for the treatment of hypertension in humans. ACE inhibitors are also employed for the treatment of heart conditions such as angina.

Certain ACE inhibitors are susceptible to certain types of degradation, such as cyclization, hydrolysis and oxidation.
Specifically, the sodium salt of Fosinopril, (4S)-4-cyclohexyl-1-{[[(RS)-1-hydroxy-2-methylpropoxy] (4-phenylbutyl) phosphinyl] acetyl}-L-proline, propionate (ester) sodium salt, is a well known drug indicated mainly for the treatment of hypertension and for the treatment of heart failure, usually in combination with a diuretic agent. Fosinopril is the ester prodrug of an angiotensin converting enzyme (ACE) inhibitor, fosinoprilat, and is converted in vivo by hydrolysis of the diester side-chain into the active component. Fosinopril is unstable, due to the fact that it has an ester (-CO-O-) and a phosphoester (=PO-O-) bond that are susceptible to hydrolytic degradation. Thus, the drug effectiveness of the compositions containing fosinopril or salts thereof can be reduced.

Moreover, Trandolapril, chemically known as (2S, 3aR, 7aS) -1-[(S) -N -[(S)-1-Carboxy -3-phenylpropyl]alanyl] hexahydro-2-indo-linecarboxylic acid, 1-ethyl ester, is an ethyl ester prodrug of a nonsulfhydryl angiotensin converting enzyme (ACE) inhibitor, namely the trandolaprilat, and is susceptible to hydrolysis of the side-chain ester group and/or cyclization via internal nucleophilic reaction to form substituted *diketopiperazines*.
The degradation of the active ingredient results in reduced drug effectiveness and treatment failure.

Furthermore, the stability of pharmaceutical compositions containing an ACE Inhibitor, and in particular, Fosinopril sodium or Trandolapril or salts thereof can also be influenced by the selection of the excipients.

Moreover, the poor flow properties of said ACE inhibitor, and in particular, Fosinopril sodium, or Trandolapril or salts thereof may also generate difficulties when it has to be formulated in dosage forms suitable for oral or sub-lingual administration such as tablets, capsules, caplets, sachets or other solid dosage forms, thus limiting the choices of the excipients that can really be used.
The bioavailability and the release rate of the pharmaceutical dosage can also be enhanced by the selection of the excipients.

Various methods are already known for the industrial preparation of oral dosage forms comprising an ACE inhibitor e.g. Fosinopril, or Trandolapril or salts thereof, as an active ingredient due to its useful antihypertensive properties. However, the prior art has encountered substantial difficulties in the production of the oral solid formulations of a desirable stability due to the degradation of said active ingredient.

EP 0 280 999 discloses a pharmaceutical composition which comprises an ACE inhibitor which is susceptible to cyclization, hydrolysis and/or discoloration, such as quinapril, enalapril and indolapril, and an amount of a stabilizer component suitable to retard cyclization, hydrolysis and/or discoloration, such as an alkaline stabilizer together with saccharides.
Furthermore, in EP 0 264 888 is disclosed an alternative stabilizing process for an ACE inhibitor using an ascorbic acid-containing stabilizer.
EP 0 408 273 proposes the use of sodium stearyl fumarate or hydrogenated vegetable oil as a lubricant in order to improve the stability of tablets containing Fosinopril sodium. Moreover, US 2002 131 999 discloses an alternative lubricant for Fosinopril sodium tablets, such as stearic acid and zinc stearate, which are more effective and less expensive

Although each of the above patents represents an attempt to overcome the instability problems associated with pharmaceuticals compositions comprising an ACE inhibitor, there still exists a need for improving the stability of such pharmaceutical compositions.

### SUMMARY OF THE INVENTION

It is, therefore, an object of the present invention to provide an improved solid dosage formulation for oral or sub-lingual administration containing an ACE inhibitor, and in particular, Fosinopril or Trandolapril or salts thereof as an active ingredient, which overcomes the deficiencies of the prior art and avoids the degradation of the active ingredient.

Another aspect of the present invention is to provide a solid dosage formulation for oral or sub-lingual administration containing an ACE inhibitor, and in particular, Fosinopril or Trandolapril or salts thereof as an active ingredient, which is bioavailable and effective with sufficient self-life and good pharmacotechnical properties.

Moreover, another aspect of the present invention is to provide a solid dosage formulation for oral or sub-lingual administration containing an ACE inhibitor, and in particular, Fosinopril or Trandolapril or salts thereof as an active ingredient, which can be prepared in dosage forms of different strength by proportionally adjusting the quantities of the excipients and the active ingredient, thereby providing a pharmacotechnical linearity, without affecting the dissolution profile and bioavailability of the active ingredient.

A further aspect of the present invention is to provide a method for the preparation of a stable solid dosage formulation for oral or sub-lingual administration containing an ACE inhibitor, and in particular, Fosinopril or Trandolapril or salts thereof as an active ingredient, thereby stabilizing said active ingredient and improving the flow properties and the pharmacotechnical characteristics of said composition.

Still, another aspect of the present invention is to provide a method of improving the flow properties of a solid dosage formulation for oral or sub-lingual administration containing an ACE inhibitor, and in particular, Fosinopril, Trandolapril or salts thereof as an active ingredient.

Another aspect of the present invention is to provide a method to use inorganic silica polymer such as Dimethicone in order to improve the flow properties of an ACE inhibitor e.g. Fosinopril or Trandolapril or salts thereof, to improve the pharmacotechnical characteristics of an ACE inhibitor or a salt thereof containing composition, to stabilize an ACE inhibitor or a salt thereof in the manufacture of a pharmaceutical composition containing an ACE inhibitor or a salt thereof.

In accordance with the above aspects of the present invention, a pharmaceutical composition for oral or sub-lingual administration is provided comprising an ACE inhibitor or a pharmaceutical acceptable salt thereof as an active ingredient, and an effective amount of inorganic silica polymer such as Dimethicone as a stabilizer to inhibit cyclization and/or hydrolysis and/or oxidation.

According to another embodiment of the present invention, a process for the preparation of solid dosage forms for oral or sub-lingual administration such as tablets, capsules and sachets containing an ACE inhibitor or a pharmaceutical acceptable salt thereof as an active ingredient is provided, which comprises:
- Forming a homogenous mixture by mixing the total quantity of said active ingredient with a total quantity of inorganic silica polymer such as Dimethicone as a stabilizer to inhibit cyclization and/or hydrolysis and/or oxidation;
- Sieving the above mixture through a sieve;
- Adding to the sieved mixture the total quantities of at least one optionally excipient such as a binder, a diluent, a disintegrant, a lubricant and/or a glidant and mixing until uniform, and
- Formulating the resulting mixture in a solid dosage form either by compressing it into a desired tablet form or by filling capsules or sachets.

Alternative processes for the preparation of the pharmaceutical composition according to the present invention are also defined in independent claims 14 and 15.

Further preferred embodiments of the present invention are defined in dependent claims 2 to 12 and 16 to 18.
Other objects and advantages of the present invention will become apparent to those skilled in the art in view of the following detailed description.

### BRIEF DESCRIPTION OF THE DRAWINGS

Fig. 1 shows dissolution profile of 20 mg tablets Fosinopril according to the present invention.
Fig. 2 shows dissolution profile of 4 mg capsules Trandolapril according to the present invention.

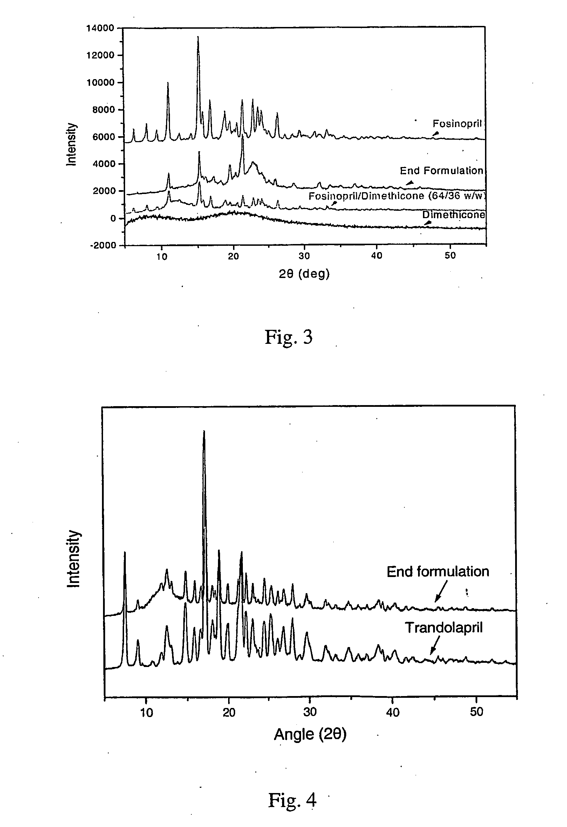

Fig. 3 shows X-RD spectrum of Fosinopril, Dimethicone, a mixture of Fosinopril /Dimethicone and a finished product according to the present invention directly after preparation.
Fig. 4 shows X-RD spectrum of Trandolapril and finished product according to the present invention directly after preparation.

### DETAILED DESCRIPTION OF THE INVENTION

For the purposes of the present invention, a pharmaceutical composition comprising an active ingredient (ACE inhibitor e.g., Fosinopril or Trandolapril or salts thereof) is considered to be "stable" if said ingredient degradates less or more slowly than it does on its own and/or in known pharmaceutical compositions.
An excipient is considered to be "incompatible" with an active ingredient (ACE inhibitor e.g., Fosinopril or Trandolapril or salts thereof) if it promotes the degradation of said active ingredient that is to say, if said active ingredient (ACE inhibitor e.g., Fosinopril or Trandolapril or salts thereof) degrades more or faster in the presence of said excipient when compared with the degradation of said active ingredient (ACE inhibitor e.g., Fosinopril, Trandolapril or salts thereof) on its own. The terms "incompatibility", "compatible" and "compatibility" are defined accordingly.
The active ingredient (ACE inhibitor e.g., Fosinopril or Trandolapril or salts thereof) contained in a dosage form is "bioavailable", if when administered in a dosage form is released from the dosage form, absorbed and reaches, at least the same, concentration levels in plasma as any of the marketed products containing the same quantity of the same active ingredient and intended for the same use.

Although the pharmaceutical composition may be in various forms, the preferred solid forms are tablets, capsules and caplets.

The improved solid pharmaceutical composition of the present invention is characterized by physicochemical properties suitable for the tablet formulation by direct compression, the adequate release rate of the active ingredient (ACE inhibitor e.g., Fosinopril or Trandolapril or salts thereof) and the storage stability, by employing excipients practically devoiding the tendency to interact with the active ingredient, and possessing good compressibility properties.

As already mentioned certain ACE inhibitors, such as Fosinopril or Trandolapril or salts thereof, are susceptible to degradation / hydrolysis and their tendency gets stronger when they are formulated and mixed with excipients or additional active ingredients such as diuretics, calcium channel blockers, beta-blockers and the like.
Moreover, said ACE inhibitors, specifically Fosinopril or salts thereof, have a relative low bulk density, poor flow properties and stick to metal surfaces during tableting. It is, therefore, necessary to employ at least a lubricant in the tablet formulation of said compositions, in order to reduce the friction during tablet compression. The lubricant deforms easily when sheared between two surfaces and, hence, when interposed between the tablet and the die wall, provides a readily deformable film that eliminates the friction between the compressed tablet and the die, so that the tablet can be removed from the die without damage.
In addition, the inclusion of a glidant is also necessary, in order to improve the flow properties for sufficient and uniform die filling. This is achieved as the glidant lodges in the irregularities of the granule surface, forming a more rounded structure and thus reducing interparticulate friction.
It must be stressed that the functions of a glidant and a lubricant in formulation process are totally different. A few materials e.g. talc, can act, at the same time, as glidant and lubricant, but usually two different excipients are required.
One of the main disadvantages of the ACE inhibitors, especially Fosinopril, is the fact that, they are incompatible with the most commonly used lubricants and glidants such as Magnesium Stearate and Silicon dioxide (colloidal).

Moreover, the lubricant and the glidant should be very carefully selected because some of them are very hydrophobic and affect negatively disintegration and dissolution while has been shown to cause bioavailability problems. The manufacturing process should also be very carefully determined because relatively high concentrations of lubricant and/or glidant reduce crashing strength and increase disintegration time especially when associated with prolonged mixing times.
Furthermore, it is already known either to include alternative excipients as lubricants with or without stabilizing agents, or to use more complicated formulations and/or manufacturing processes.

Tablets comprising Fosinopril sodium and Magnesium Stearate as the lubricant are relatively unstable. Furthermore, the use of Hydrogenated Vegetable Oil can cause processing problems of sticking to the punches during long tableting runs, Sodium Stearyl Fumarate is much more expensive than other commonly used lubricants and causes similar tablet strength reduction and prolongation of disintegration time. Moreover, Glyceryl Dibehenate has the least anti-adherent effect and it is required at higher levels than, for example, Magnesium Stearate or Sodium Stearyl Fumarate, for effective lubrication, and besides, it has a not negligible retardant effect on dissolution rate.

In addition, the compositions of the present invention contain, optionally, at least one additional active ingredient.

It has been surprisingly found that the object of the present invention is achieved by employing a low and medium density inorganic silica polymer such as Dimethicone as a stabilizer, while its nominal viscosity may range from 50 cSt to 1000 cSt. Dimethicone is a known excipient used in cosmetic and pharmaceutical formulations as an antifoaming agent and emollient.

Dimethicone is a linear polydimethysiloxane polymer with a degree of polymerisation in the range of n=20 to 400 and a nominal kinematic viscosity in the range of 20 to 1300 mm²/s (from 20cSt to 1300cSt). Dimethicone is commercially available under different grades of density and various viscosities. Moreover, dimethicone is a hydrophobic oily substance, practically insoluble in water, resistant to heat and stable during storage.

When dimethicone is incorporated in a pharmaceutical composition according to the present invention it is adsorbed by the crystal or amorphous particles of the active ingredient resulting in one-phase system. In said system, Dimethicone and the active ingredient are in contact in a molecular basis. Said one-phase system protects the active ingredient from oxidation and/or acid hydrolysis and/or cyclization. Thus, Dimethicone serves as a protective barrier, isolating the active ingredient against humidity and/or air oxygen. Moreover, the crystals of the active ingredient become soften and more regular in shape leading in improved compressibility and better flowability. The protection of the active ingredient may be partially attributed to the hydrophobic character of Dimethicone. This, however, unexpectedly does not affect the dissolution rate of the active ingredient as it is used in such a proportion that its emulsifying properties prevail and therefore excellent bioavailability is achieved.

It has been confirmed through several tests such as FT-IR spectra analysis, DSC thermal analysis, X-RD analysis, and SEM anlysis, that Fosinopril or Trandolapril or salts thereof and Dimethicone according to the formulation of the present invention, are chemically inert and, thus, there is no chemical interaction between the two substances and all characteristic absorption peaks and bands of Fosinopril or Trandolapril or salts thereof are present in the mixture, as well.

Furthermore, the active ingredient remained chemically untouched, showing the same melting point whether it is analysed in the mixture or by itself. In addition, the X-RD analysis showed that the crystal of Fosinopril/ Trandolapril or salts thereof remained invariable after suspended in Dimethicone. Besides SEM analysis revealed that the shape of the crystals did not change.

Specific tablet characteristics such as stability, resistance to crashing and friability are much more improved with the use of Dimethicone. Furthermore, Dimethicone is colorless, odorless and, thus, also possesses good organoleptic characteristics.

A mixture of the active ingredient (ACE inhibitor, especially Fosinopril or Trandolapril or salts thereof) with a suitable amount of inorganic silica polymer such as Dimethicone is formed, and subsequently admixed to complete homogeneity. After sieving the mixture, any optional additional excipient is then added. The composition is then mixed until uniform. The resulting composition may then be compressed.
The bulk density of the above composition is effectively reduced and the flow is impressively improved to such a degree that a glidant is not required any more, while, unexpectedly, the composition does not adhere to the tableting machine and uniform die filling is accomplished.
Moreover, any excipient may optionally be added to the above composition, provided that they are compatible with the active ingredient of the composition, in order to overcome problems associated with the poor flow properties and unfavorable pharmacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms for oral or sub-lingual administration of an active ingredient having stability problems especially related with oxygen and/or humidity of the atmosphere.
Another essential advantage of the present invention is that the solid dosage form according to the present invention ensures excellent bioavailability of the active ingredient. Furthermore, it is possible to prepare dosage forms of different strength using appropriate quantity of the same composition, thereby limiting the cost of production and minimizing the number, and consequently the cost, of clinical studies required for the approval of the product by the authorities.
The manufacturing process for preparation according to the present invention is simpler and inexpensive in comparison to any other conventional method.

Therefore, in a first embodiment, the present invention provides a pharmaceutical composition comprising from about 0.5% to 50% by weight of Fosinopril or Trandolapril or salts thereof and from about 0.1 % to 25% by weight of Dimethicone. The weight ratio of the Fosinopril/ Trandolapril or salts thereof to Dimethicone is preferably 500:1 to 1:50 and more preferably 200:7.5 to 7.5:80.

Preferred pharmaceutical compositions according to the present invention comprise approximately 0.5% to 40%, more preferably 0.75% to 25% and most preferably 0.75% to 20% by weight of Fosinopril or Trandolapril or salts thereof.

More preferred pharmaceutical compositions according to the present invention comprise approximately 0.2% to 20%, more preferably 0.5% to 15% and most preferably 0.75% to 10% by weight of Dimethicone.
The preferred pharmaceutical compositions are in the form of solid dosage forms for oral or sub-lingual administration such as tablets, capsules, caplets, troches, pastilles, pills, lozenges and the like, in all shapes and sizes, coated or uncoated.
All percentages stated herein are weight percentages based on total composition weight, unless otherwise stated.

Another embodiment of the present invention is the use of the direct compression process for the preparation of solid dosage forms such as tablets containing Fosinopril or Trandilapril or salts thereof, which is one of the most economical methods.

The direct compression process of the present invention for the preparation of solid dosage forms for oral or sub-lingual administration such as tablets containing ACE inhibitor such as Fosinopril or Trandolapril or salts thereof as an active ingredient comprises:
- Forming a homogenous mixture by mixing the total quantity of the active ingredient with the total quantity of a suitable amount of inorganic silica polymer such as Dimethicone as a stabilizer to inhibit cyclization and/or hydrolysis and/or oxidation;
- Sieving the above mixture through a sieve, and subsequently;
- Adding to the sieved mixture the total quantities of at least one optionally excipient such as a binder, a diluent, a filler, a disintegrant, a lubricant and/or a glidant and mixing until uniform, and
- Formulating the resulting mixture in a solid dosage form either by compressing it into a desired tablet form or by filling capsules or sachets.

Alternatively, a blend of the total quantity of the active ingredient (ACE inhibitor, such as Fosinopril or Trandolapril or salts thereof) and/or the total batch quantity or a portion thereof of an optional diluent, and/or the total batch quantity or a portion thereof of an optional filler is formed and subsequently is admixed with the total batch quantity of an effective amount of Dimethicone to complete homogeneity. Then any other optional auxiliary excipient may be added.

Further, in the sieved mixture mentioned above, it may optionally be added at least one additional active ingredient such as diuretics, channel blockers, beta-blockers, and the like.
The final mixture of the composition can be compressed into tablets or caplets, filled into capsules, or processed into another solid form.

Alternatively, a wet granulation process may also be used for the preparation of the pharmaceutical composition of the present invention. Said wet granulation process comprises:
- forming a first blend of the total quantity of the active ingredient (ACE inhibitor, such as Fosinopril or Trandolapril or salts thereof) with an effective amount of inorganic silica polymer, such as Dimethicone, as a stabilizer and/or the total batch quantity or a portion thereof of an optional diluent, and/or the total batch quantity or a portion thereof of an optional filler;
- mixing, optionally, an additional active ingredient with the total quantity of at least one optional excipient such as a binder, a disintegrant, a lubricant and/or a glidant until uniform and wet granulating by the addition of a water-free granulating medium (i.e. absolute ethanol, acetone);
- drying the wetted mass,
- sieving the dried material to achieve the desired granule size
- adding and mixing the dried material with the first blend and
- formulating the resulting mixture in a solid dosage form either by compressing it into a desired tablet form or by filling capsules or sachets.

The pharmaceutical compositions of the present invention are characterized by excellent pharmacotechnical properties, such as homogeneity, flowability and compressibility. Thanks to these properties, the solid dosage forms prepared by the above process exhibit excellent technical characteristics including disintegration time, dissolution rate, hardness, resistance to crashing, friability and stability, as better illustrated by the following measurements during the stage of the development of the products.
Namely, the pure pharmaceutical substance showed acceptable flowability and compressibility with a mean Carr's Index of 30%, whilst the angle of repose was found to be about 30°.

All the above results indicate that Fosinopril sodium and Trandolapril have good to moderate flow properties, which can be improved by the addition of suitable ingredients in order to select a direct compression process for the final formulation.

One of the most critical pharmacotechnical tests, is the Dissolution test as it is strongly correlated with the bioavailability of the product. For the dissolution method a Paddle Apparatus was used 50rpm, 37°C, time 30min, while as a dissolution medium 900ml of H₂O was used.
The dissolution profile of the composition showed a fast release profile, with more than 70% release in 5min, more than 80% in 15min, while the fmal release is about 100% in 30 minutes.

All the above mentioned characteristics were also investigated for a formulation of 10mg and 20mg strength equivalent weight of Fosinopril per tablet, and for a formulation of 0.5mg, 1mg, 2mg and 4mg strength equivalent weight of Trandolapril per capsule.

In order to prepare small, smoothly swallowable tablets the same mixture of excipients as for the strength of 10mg were used.

The most preferable compositions described below were investigated for their scalability, while a process validation was performed in order to prove the repeatability and accuracy of the manufacturing process and the proposed formulations. For the above tests 3 batches per strength were used.

The validation process showed that the compositions and the manufacturing process are suitable in order to provide a repeatable and high quality product.
Namely, the appearance was found to be acceptable in all cases. The disintegration time was less than 8 min, the dissolution was found to be over 90% in 30 minutes, whilst the Assay was between 99 and 102%.
No degradation products were observed during and after the procedure.

One of the main objects of the present invention was to prepare a product with acceptable stability. For this reason, 3 batches of all strength were exposed to normal and accelerated stability studies according to the current ICH guidelines.

The following compositions were used per strength.

| ***Strength*** | **10mg** | **20mg** |
|---|---|---|
| ***Ingredients*** | **mg per tablet** | |
| Fosinopril Sodium | 10.00 | 20.00 |
| Dimethicone | 2.50 | 5.00 |
| Crospovidone | 5.60 | 11.20 |
| Micr. Cellulose | 80.90 | 161.80 |
| Lactose | 45.00 | 90.00 |
| Starch 1500 | 16.00 | 32.00 |

The tablets were packed in into containers impervious to water vapor e.g. PVC/PVDC and stored in appropriate stability chambers at a temperature of 25°C±2°C and relative humidity of 60%±5% for normal conditions and at a temperature of 40°C and relative humidity of 75% for accelerated conditions. The tablets were tested in predetermined time intervals.

| ***Strength*** | **0.5mg** | **1mg** | **2mg** | **4mg** |
|---|---|---|---|---|
| ***Ingredients*** | **mg per capsule** | | | |
| **Trandolapril** | 0.5 | 1.0 | 2.0 | 4.0 |
| Dimethicone | 2.0 | 2.0 | 2.0 | 2.0 |
| MCC | 24.0 | 24.0 | 24.0 | 24.0 |
| Lactose monohydrate | 24.0 | 24.0 | 24.0 | 24.0 |
| Starch 1500 | 47.5 | 47.5 | 47.5 | 47.5 |
| Aerosil | 1.0 | 1.0 | 1.0 | 1.0 |
| Mg stearate | 1.0 | 1.0 | 1.0 | 1.0 |

The frequency of the testing, the specific tests and results indicated for each batch are described in the stability table (TABLE 1 and 2).

The results show a good stability of the product and compatibility between the drug substance and the excipients proposed by the present invention. The excellent results regarding the physicochemical characteristics, the excellent stability of the product as well as the simple and economic manufacturing process indicate the advantages of the present invention relative to the commonly used methods and excipients for the formulation of Fosinopril sodium and Trandolapril.

**TABLE 1: STABILITY AT 40 °C ± 2°C TEMPERATURE AND 75±5% RELATIVE HUMIDITY**

| **Fosinopril tablets 20 mg** | | | | |
|---|---|---|---|---|
| ***Control*** | ***Specification*** | ***Time In Months*** | | |
| | | **0** | **3** | **6** |
| Appearance | *White round tablets* | No change | No change | No change |
| Assay (by HPLC) | *95-105%* | 100.9% | 100.5% | 100.2% |
| Disintegration Time | *Max 15 min in water at 37°C* | 2'10"-2'30" | 2'15"-2'30" | 2'10"-2'35" |
| Dissolution (Paddles, 900ml water, 50 rpm) | *Each tablet >85% of the stated amount in 30 min* | 101.5% | 99.4% | 100.7% |
| **Related substances** | | | | |
| Impurity A+G | NMT 0.20% | 0.04 | 0.07 | 0.11 |
| Impurity C+H | NMT 0.50% | 0.14 | 0.16 | 0.15 |
| Impurity B | NMT 0.20% | 0.07 | 0.09 | 0.10 |
| Impurity F | NMT 0.20% | 0.08 | 0.11 | 0.12 |
| Total Unknown | NMT 0.20% | - | - | - |
| TOTAL | NMT 1.0% | **0.33** | **0.43** | **0.48** |

**TABLE 2: STABILITY AT 40°C ± 2°C TEMPERATURE AND 75±5% RELATIVE HUMIDITY**

| **Trandolapril capsules 4 mg** | | | | |
|---|---|---|---|---|
| ***Control*** | ***Specification*** | ***Time In Months*** | | |
| | | **0** | **3** | **6** |
| Appearance | *Light-scarlet- light-scarlet No2 capsule* | No change | No change | No change |
| Assay (by HPLC) | *95-105%* | 101.6% | 100.4% | 100.6% |
| Disintegration Time | *Max 15 min in water at 37°C* | 5'10"-6'30" | 5' 15''-6'20'' | 5' 08"-6'26" |
| Dissolution (Paddles, 900ml water, 50 rpm) | *Each tablet >85% of the stated amount in 30min* | 101.3% | 100.4% | 100.0% |
| **Related substances** | | | | |
| Impurity ECAPPA | *NMT 0.20%* | 0.01% | 0.01% | 0.01% |
| *Trandolaprilat* | *NMT 0.20%* | 0.03% | 0.08% | 0.12% |
| Isomer | *NMT 0.20%* | ND | ND | ND |
| Cyclohexyl Analogue | *NMT 0.20%* | 0.03% | 0.03% | 0.03% |
| *Diketopiperazine* | *NMT 0.20%* | ND | 0.08% | 0.10% |
| Octahydroindole-2- | *NMT 0.50%* | ND | ND | ND |
| carboxylic acid | | | | |
| Single unknown | *NMT 0.50%* | ND | 0.03% | 0.01% |
| | | | | |
| TOTAL | ***NMT 1.7 %*** | **0.07%** | **0.24%** | **0.27%** |

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions.

Such ingredients include, but are not limited to, diluents, binders, compression aids, disintegrants, glidants, lubricants, flavors, water scavengers, colorants, sweetener, coating agents and preservatives.

The optional excipients must be compatible with the ACE inhibitor or the salt thereof so that it does not interfere with it in the composition.
Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch, sucrose.
Disintegrants may be, for example, alginic acid, carbon dioxide, carboxymethylcellulose calcium, carboxymethylcellulose sodium, microcrystalline cellulose, powdered cellulose, croscarmelose sodium, crospovidone, sodium docusate, guar gum, hydroxypropyl cellulose, methylcellulose, polacrilin potassium, poloxamer, povidone, sodium alginate, sodium glycine carbonate, sodium laulyl sulfate, sodium starch glycolate, starch, pregelatinized starch.
Glidants may be, for example, calcium silicate, powdered cellulose, starch, talc, lubricants e.g. polyethylene glycol 4000, polyethylene glycol 6000, sodium lauryl sulfate, starch, talc.

Moreover, the additional active ingredient may be selected from a wide range of diuretics, such as hydrochlorothiazide, or calcium channel blockers such as verapamil or beta-blockers, and the like.

Still another embodiment of the present invention is the use of inorganic silica polymer, such as Dimethicone, as an agent to improve flow properties of ACE inhibitors/Fosinopril, Trandolapril and/or to prevent sticking to parts of the processing machines, for example tableting machine and/or to protect and stabilize cyclization and/or hydrolysis and/or oxidation susceptible pharmaceutical substances.

The following examples illustrate preferred embodiments in accordance with the present invention without limiting the scope or spirit of the invention:

### EXAMPLES

### Example 1:

**Tablet of 20 mg Fosinopril**

| Ingredients | Per Tablet |
|---|---|
| Fosinopril | 20.00 mg |
| Micr. Cellulose | 149.00 mg |
| Povidone | 7.50 mg |
| Crospovidone | 12.00 mg |
| Starch pregelatinized | 25.50 mg |
| Lactose monohydrate | 90.00 mg |
| Dimethicone | 16.00 mg |
| Total weight: | 320.00 mg |

20000 tablets of the above formulation were prepared according to the following manufacturing process: Fosinopril (400 g) and Dimethicone (320 g) were admixed to complete homogeneity. The above mixture was passed through a sieve. The sieved mixture was then mixed with all the other excipients (Micr. Cellulose 2980.0 g, Povidone 150.0 g, Crospovidone 240.0 g, Starch pregelatinized, 510.0 g, Lactose monohydrate, 1800.0 g) for about 15 minutes. The final mixture was then compressed directly into tablets in a tableting machine with round punches of a 10mm diameter. The tablets were packed into blisters of PVC-PVDC.

The bulk mixture showed satisfactory flow and could also be filled into capsules or sachets or compressed into tablets. The later solution was selected and the produced tablets were tested for hardness, friability, disintegration, and water content. All tests were performed according to European Pharmacopoeia 5.1 and were well within the specifications. Dissolution test in 900 ml water, 50 rpm Paddle Apparatus showed more than 70% dissolved in 10 min and more than 80% in 15 min.

### Example 2:

**Capsule of 20 mg Fosinopril**

| Ingredients | Per Capsule |
|---|---|
| Fosinopril | 20.00 mg |
| Micr. Cellulose | 156.80 mg |
| Crospovidone | 12.00 mg |
| Starch pregelatinized | 30.00 mg |
| Lactose monohydrate | 90.00 mg |
| Dimethicone | 11.20 mg |
| Total weight: | 320.00 mg |

20000 capsules were prepared using the procedure of Example 1( Fosinopril 400.0 g, Micr. Cellulose 3136.0 g, Crospovidone 240.0 g, Starch pregelatinized 600.0 g, Lactose monohydrate 1800.0 g, Dimethicone 224.0 g).
The produced bulk mixture showed slightly better flow properties and could be formulated either as capsules or sachets or compressed into tablets. Capsules were produced and tested for content uniformity, disintegration, water content and dissolution proving that they are meeting the specifications.

### Example 3

**Tablet of 20 mg Fosinopril**

| Ingredients | Per Tablet |
|---|---|
| Fosinopril | 20.00 mg |
| Micr. Cellulose | 161.80 mg |
| Crospovidone | 11.20 mg |
| Starch pregelatinized | 32.00 mg |
| Lactose monohydrate | 90.00 mg |
| Dimethicone ABIL 350 | 5.00 mg |
| Total weight: | 320.00 mg |

20000 tablets were prepared using the procedure of Example 1 (Fosinopril 400.0 g, Micr. Cellulose 3236.0 g, Crospovidone 224.0 g, Starch pregelatinized 640.0 g, Lactose monohydrate 1800.0 g, Dimethicone ABIL 350 100.0 g).
The produced bulk mixture showed greatly improved flow properties (30% decrease of Carr index) and could be formulated either as capsules or sachets or compressed into tablets. From this bulk, tablets weighting 320mg containing 20 mg Fosinopril were produced and tested for hardness, friability, disintegration, and water content and results were well within the specifications. Furthermore dissolution in 900 ml water, 50 rpm Paddle Apparatus showed more than 70% dissolved in 5 min and more than 85% in 15 min.

### Example 4

**Tablet of 10 mg Fosinopril**

| Ingredients | Per Tablet |
|---|---|
| Fosinopril | 10.00 mg |
| Micr. Cellulose | 80.90 mg |
| Crospovidone | 5.60 mg |
| Starch pregelatinized | 16.00 mg |
| Lactose monohydrate | 45.00 mg |
| Dimethicone ABIL 350 | 2.50 mg |
| Total weight: | 160.00 mg |

20000 tablets were prepared using the procedure of Example 1 (Fosinopril 200.0 g, Micr. Cellulose 1618.0 g, Crospovidone 112.0 g, Starch pregelatinized 320.0 g, Lactose monohydrate 900.0 g, Dimethicone ABIL 350 50.0 g).

Tablets weighing 160mg containing 10 mg Fosinopril were produced and tested for hardness, friability, disintegration, and water content and the results were well within the specifications. Dissolution test performed in 900 ml water, 50 rpm Paddle Apparatus showed more than 70% dissolved in 5 min and more than 85% in 15 min.
These results demonstrate that dissolution profile remains unaffected besides the lower strength, proving that pharmacotechnical linearity i.e. proportional change in amount of excipients and active and total weight of the dosage form is achieved.

### Example 5 :

**Tablet of 20 mg Fosinopril**

| Ingredients | Per Tablet |
|---|---|
| Fosinopril | 20.00 mg |
| Micr. Cellulose | 150.00 mg |
| Crospovidone | 15.00 mg |
| Starch pregelatinized | 30.00 mg |
| Lactose monohydrate | 90.00 mg |
| Dimethicone | 15.00 mg |
| Total weight: | 320.00 mg |

20000 tablets of the above formulation were prepared according to the following manufacturing process: Fosinopril (400 g) and 1/3 of the batch quantity of the Micr. Cellulose (3000.0 g) were added in Dimethicone (300 g) and the formulation was admixed to complete homogeneity. The above mixture was passed through a sieve. The sieved mixture was then mixed with all the other excipients (2/3 of the batch quantity of the Micr. Cellulose 3000.0 g, Crospovidone 300.0 g, Starch pregelatinized, 600.0 g, Lactose monohydrate, 1800.0 g) for about 15 minutes. The final mixture was then compressed into tablets in a tableting machine with round punches.

The bulk mixture showed satisfactory flow and could be simply filled into capsules or sachets or compressed into tablets. In fact it was divided in two portions the first of which was compressed into tablets, while the second was filled in capsules. Both tablets and capsules were tested for water content, disintegration, dissolution and gave quite satisfactory results. Tablets were further tested for hardness and friability and found to comply with the specifications.

### Example 6 :

**Tablet of 20 mg Fosinopril**

| Ingredients | Per Tablet |
|---|---|
| Fosinopril | 20.00 mg |
| Hydrochlorothiazide | 12.50 mg |
| Micr. Cellulose | 135.00 mg |
| Hydroxypropyl Cellulose | 18.00 mg |
| Dicalcium Phosphate | 30.00 mg |
| Starch pregelatinized | 21.00 mg |
| Lactose monohydrate | 39.50 mg |
| Primogel | 29.00 mg |
| Dimethicone | 10.00 mg |
| Total weight: | 315.00 mg |

20000 tablets of the above formulation were prepared according to the following manufacturing process: Fosinopril (400 g) and Dimethicone (200 g) were admixed to complete homogeneity. The above mixture was passed through a sieve. The sieved mixture was then mixed with Hydrochlorothiazide (250.0 g) and all the other excipients (Micr. Cellulose 2700.0 g, Hydroxypropyl Cellulose 360.0 g, Dicalcium Phosphate 600.0 g, Starch pregelatinized, 420.0 g, Lactose monohydrate, 790.0 g, Primojel, 580.0 g) for about 15 minutes. The final mixture was then compressed directly into tablets in a tableting machine with round punches.

### Example 7 :

**Capsule of 0.5 mg Trandolapril**

| Ingredients | Per Capsule |
|---|---|
| Trandolapril | 0.50 mg |
| Dimethicone | 2.00 mg |
| Micr. Cellulose | 24.00 mg |
| Lactose monohydrate | 24.00 mg |
| Starch 1500 | 47.50 mg |
| Aerosil | 1.00 mg |
| Mg stearate | 1.00 mg |
| Total weight: | 100.00 mg |

### Example 8 :

**Capsule of 1.0 mg Trandolapril**

| Ingredients | Per Capsule |
|---|---|
| Trandolapril | 1.00 mg |
| Dimethicone | 2.00 mg |
| Micr. Cellulose | 24.00 mg |
| Lactose monohydrate | 24.00 mg |
| Starch 1500 | 47.50 mg |
| Aerosil | 1.00 mg |
| Mg stearate | 1.00 mg |
| Total weight: | 100.50 mg |

### Example 9 :

**Capsule of 2.0 mg Trandolapril**

| Ingredients | Per Capsule |
|---|---|
| Trandolapril | 2.00 mg |
| Dimethicone | 2.00 mg |
| Micr. Cellulose | 24.00 mg |
| Lactose monohydrate | 24.00 mg |
| Starch 1500 | 47.50 mg |
| Aerosil | 1.00 mg |
| Mg stearate | 1.00 mg |
| Total weight: | 101.50 mg |

### Example 10 :

**Capsule of 4.0 mg Trandolapril**

| Ingredients | Per Capsule |
|---|---|
| Trandolapril | 4.00 mg |
| Dimethicone | 2.00 mg |
| Micr. Cellulose | 24.00 mg |
| Lactose monohydrate | 24.00 mg |
| Starch 1500 | 47.50 mg |
| Aerosil | 1.00 mg |
| Mg stearate | 1.00 mg |
| Total weight: | 103.50 mg |

Capsules of the formulations of the Examples 7 to 10 were prepared according to the following manufacturing process: Trandolapril and Aerosil were accurately added in Dimethicone and the formulation was admixed to complete homogeneity. The above mixture was passed through a sieve: The sieved mixture was then mixed with all the other excipients (Micr. Cellulose, Starch 1500, Lactose monohydrate, Magnesium stearate) for about 15 minutes.

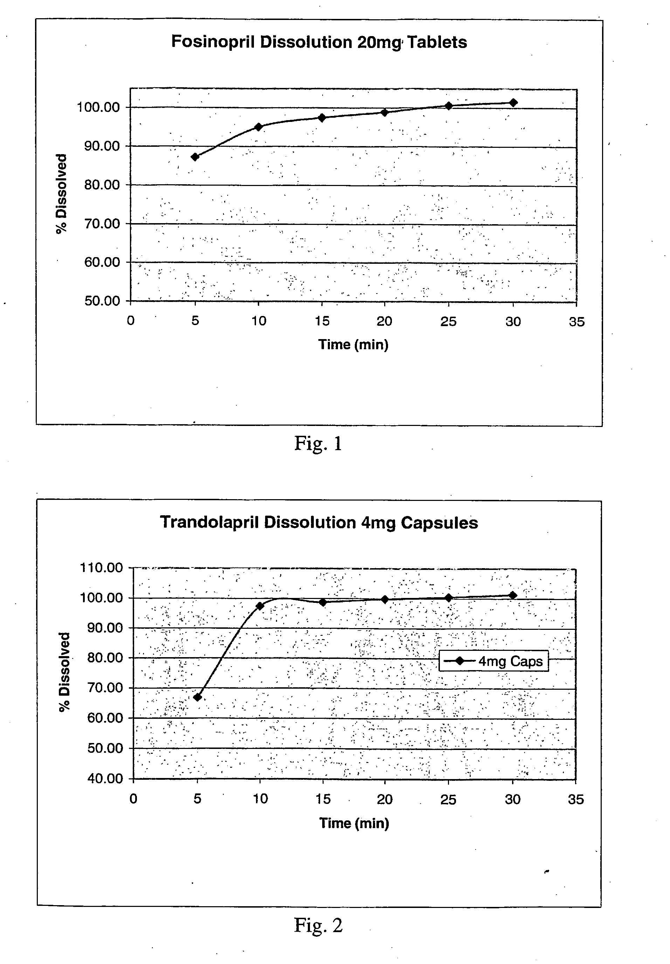

The produced bulk mixture showed slightly better flow properties and could be formulated either as capsules or sachets or compressed into tablets. Capsules were produced and tested for content uniformity, disintegration, water content and dissolution proving that they are meeting the specifications (see Fig. 2).

To provide a better homogeneity of the Trandolapril in Dimethicone, small amounts of Aerosil is added to the formulation, which increases the suspendibility of the active ingredient in Dimethicone.

### Example 11 :

**Capsule of 2.0 mg Trandolapril**

| Ingredients | Per Capsule |
|---|---|
| Trandolapril | 2.00 mg |
| Verapamil | 180.00 mg |
| Dimethicone | 2.00 mg |
| Micr. Cellulose | 40.00 mg |
| Lactose monohydrate | 24.00 mg |
| Starch 1500 | 50.00 mg |
| Aerosil | 1.00 mg |
| Mg stearate | 1.00 mg |
| Total weight: | 300.00 mg |

Capsules of the formulations of the Examples 11 were prepared according to the following manufacturing process: Trandolapril and Aerosil were accurately added in Dimethicone and the formulation was admixed to complete homogeneity.
The above mixture was passed through a sieve. The sieved mixture was then mixed with Verapamil and all the other excipients (Micr. Cellulose, Starch 1500, Lactose monohydrate, Magnesium stearate) for about 15 minutes.

The produced bulk mixture showed satisfactory flow properties and could be formulated either as capsules or sachets or compressed into tablets.

## Claims

1. A pharmaceutical composition for oral or sub-lingual administration comprising an ACE inhibitor or a pharmaceutical acceptable salt thereof, as an active ingredient, and an effective amount of inorganic silica polymer such as Dimethicone as a stabilizer to inhibit cyclization and/or hydrolysis and/or oxidation.

2. The pharmaceutical composition according to claim 1, wherein said inorganic silica polymer is Dimethicone.

3. The pharmaceutical composition according to claim 2, wherein it comprises from 0.5 to 50% by weight of said ACE inhibitor or salt thereof, and from 0.1 to 25 % by weight of said Dimethicone.

4. The pharmaceutical composition according to claim 2, wherein the weight ratio of said ACE inhibitor or salt thereof to Dimethicone is preferably 500:1 to 1:50 and more preferably 200:7.5 to 7.5:80.

5. The pharmaceutical composition according to claim 1, wherein it comprises approximately 0.5% to 40%, more preferably 0.75% to 25% and most preferably 0.75% to 20% by weight of said ACE inhibitor or salt thereof.

6. The pharmaceutical composition according to claim 2, wherein it comprises approximately 0.2% to 20%, more preferably 0.5% to 15% and most preferably 0.75% to 10% by weight of Dimethicone.

7. The pharmaceutical composition according to any preceding claim, wherein said ACE inhibitor is Fosinopril or a salt thereof.

8. The pharmaceutical composition according to any preceding claim, wherein said ACE inhibitor is Trandolapril or a salt thereof

9. The pharmaceutical composition according to any preceding claim, wherein it further comprises at least one additional active ingredient.

10. The pharmaceutical composition according to claim 9, wherein said additional active ingredient is selected from the group of diuretics such as hydrochlorothiazide or the group of calcium channel blockers such as verapamil or the group of beta-blockers.

11. The pharmaceutical composition according to any preceding claim, wherein it further comprises at least one optionally excipient selected from the group consisting of diluents, binders, disintegrants, lubricants, and glidants.

12. The pharmaceutical composition according to any preceding claim, wherein said composition is in a solid dosage form such as a tablet, capsule or sachet comprising an active ingredient such as Fosinopril or Trandolapril or salts thereof.

13. A process for the preparation of a solid dosage form for oral or sub-lingual administration such as a tablet, capsule or sachet containing an ACE inhibitor or a pharmaceutical acceptable salt thereof as an active ingredient and an effective amount of inorganic silica polymer such as Dimethicone as a stabilizer to inhibit cyclization and/or hydrolysis and/or oxidation, which comprises:
- Forming a homogenous mixture by mixing the total quantity of said active ingredient with the total quantity of said Dimethicone;
- Sieving the above mixture through a sieve;
- Adding to the sieved mixture the total quantities of at least one optionally excipient such as a binder, a diluent, a disintegrant, a lubricant and/or a glidant and mixing until uniform, and
- Formulating the resulting mixture in a solid dosage form either by compressing it into a desired tablet form or by filling capsules or sachets.

14. A process for the preparation of a solid dosage form for oral or sub-lingual administration, such as a tablet, a capsule or a sachet, containing an ACE inhibitor or a pharmaceutical acceptable salt thereof as an active ingredient and an effective amount of inorganic silica polymer such as Dimethicone as a stabilizer to inhibit cyclization and/or hydrolysis and/or oxidation, which comprises:
- Forming a homogenous mixture by mixing the total quantity of said active ingredient and/or the total batch quantity or a portion thereof of an optional diluent, and/or the total batch quantity or a portion thereof of an optional filler;
- Screening the above mixture through a screen and subsequently admixing with the total quantity of the effective amount of Dimethicone;
- Sieving the above mixture on a sieve;
- Adding to the sieved mixture the total quantity of any other optionally excipient and mixing until uniform, and
- Formulating the resulting mixture in a solid dosage form either by compressing it into a desired tablet form or by filling capsules or sachets.

15. A process for the preparation of a solid dosage form for oral or sub-lingual administration, such as a tablet, a capsule or a sachet, containing an ACE inhibitor or a pharmaceutical acceptable salt thereof as an active ingredient and an effective amount of inorganic silica polymer such as Dimethicone as a stabilizer to inhibit cyclization and/or hydrolysis and/or oxidation, which comprises:
- forming a first blend of the total quantity of said active ingredient with an effective amount of inorganic silica polymer, such as Dimethicone, as a stabilizer and/or the total batch quantity or a portion thereof of an optional diluent, and/or the total batch quantity or a portion thereof of an optional filler;
- mixing, optionally, an additional active ingredient with the total quantity of at least one optional excipient such as a binder, a disintegrant, a lubricant and/or a glidant until uniform and wet granulating by the addition of a water-free granulating medium such as absolute ethanol, acetone or mixtures thereof;
- drying the wetted mass;
- sieving the dried material to achieve the desired granule size
- adding and mixing the dried material with the first blend and
- formulating the resulting mixture in a solid dosage form either by compressing it into a desired tablet form or by filling capsules or sachets.

16. The process according to claims 13 to 15, wherein said active ingredient is Fosinopril or salt thereof.

17. The process according to claims 13 to 15, wherein said active ingredient is Trandolapril or salt thereof.

18. The process according to claim 13 or 14, wherein in said sieved mixture it is further added at least one additional active ingredient selected from the group of diuretics such as hydrochrlorothiazide or the group of calcium channel blockers such as verapamil or the group of beta-blockers.

## Patentansprüche

1. Pharmazeutische Zusammensetzung zur oralen oder sublingualen Verabreichung, enthaltend einen ACE-Inhibitor oder ein pharmazeutisch unbedenkliches Salz davon als Wirkstoff und eine wirksame Menge eines anorganischen Siliciumdioxidpolymers wie Dimethicon als Stabilisator zum Inhibieren von Cyclisierung und/oder Hydrolyse und/oder Oxidation.

2. Pharmazeutische Zusammensetzung nach Anspruch 1, wobei es sich bei dem anorganischen Siliciumdioxidpolymer um Dimethicon handelt.

3. Pharmazeutische Zusammensetzung nach Anspruch 2, enthaltend 0,5 bis 50 Gew.-% an ACE-Inhibitor oder Salz davon und 0,1 bis 25 Gew.-% Dimethicon.

4. Pharmazeutische Zusammensetzung nach Anspruch 2, wobei das Gewichtsverhältnis von ACE-Inhibitor oder Salz davon zu Dimethicon vorzugsweise 500:1 bis 1:50 und besonders bevorzugt 200:7,5 bis 7,5:80 beträgt.

5. Pharmazeutische Zusammensetzung nach Anspruch 1, enthaltend etwa 0,5 Gew.-% bis 40 Gew.-%, besonders bevorzugt 0,75 Gew.-% bis 25 Gew.-% und ganz besonders bevorzugt 0,75 Gew.-% bis 20 Gew.-% an ACE-Inhibitor oder Salz davon.

6. Pharmazeutische Zusammensetzung nach Anspruch 2, enthaltend etwa 0,2 Gew.-% bis 20 Gew.-%, besonders bevorzugt 0,5 Gew.-% bis 15 Ges.-% und ganz besonders bevorzugt 0,75 Gew.-% bis 10 Gew.-% Dimethicon.

7. Pharmazeutische Zusammensetzung nach einem der vorhergehenden Ansprüche, wobei es sich bei dem ACE-Inhibitor um Fosinopril oder ein Salz davon handelt.

8. Pharmazeutische Zusammensetzung nach einem der vorhergehenden Ansprüche, wobei es sich bei dem ACE-Inhibitor um Trandolapril oder ein Salz davon handelt.

9. Pharmazeutische Zusammensetzung nach einem der vorhergehenden Ansprüche, weiterhin enthaltend wenigstens einen zusätzlichen Wirkstoff.

10. Pharmazeutische Zusammensetzung nach Anspruch 9, wobei der zusätzliche Wirkstoff ausgewählt ist aus der Gruppe der Diuretika wie Hydrochlorothiazid oder der Gruppe der Calciumkanalblocker wie Verapamil oder der Gruppe der Betablocker.

11. Pharmazeutische Zusammensetzung nach einem der vorhergehenden Ansprüche, weiterhin enthaltend wenigstens einen fakultativen Exzipienten ausgewählt aus der Gruppe bestehend aus Verdünnungsmitteln, Bindemitteln, Sprengmitteln, Schmiermitteln und Gleitmitteln.

12. Pharmazeutische Zusammensetzung nach einem der vorhergehenden Ansprüche, wobei die Zusammensetzung in einer festen Dosierungsform wie einer Tablette, einer Kapsel oder einem Beutel enthaltend einen Wirkstoff wie Fosinopril oder Trandolapril oder Salze davon vorliegt.

13. Verfahren zur Herstellung einer festen Dosierungsform zur oralen oder sublingualen Verabreichung wie einer Tablette, einer Kapsel oder eines Beutels enthaltend einen ACE-Inhibitor oder ein pharmazeutisch unbedenkliches Salz davon als Wirkstoff und eine wirksame Menge eines anorganischen Siliciumdioxidpolymers wie Dimethicon als Stabilisator zur Inhibierung von Cyclisierung und/oder Hydrolyse und/oder Oxidation, bei dem man:
- eine homogene Mischung bildet, indem man die Gesamtmenge an Wirkstoff mit der Gesamtmenge an Dimethicon mischt;
- die obige Mischung durch ein Sieb siebt;
- die gesiebte Mischung mit den Gesamtmengen an wenigstens einem fakultativen Exzipienten wie einem Bindemittel, einem Verdünnungsmittel, einem Sprengmittel, einem Schmiermittel und/oder einem Gleitmittel versetzt und mischt, bis man eine einheitliche Mischung erhält, und
- die erhaltene Mischung als eine feste Dosierungsform formuliert, indem man sie entweder zu einer gewünschten Tablettenform komprimiert oder indem man Kapseln oder Beutel füllt.

14. Verfahren zur Herstellung einer festen Dosierungsform zur oralen oder sublingualen Verabreichung wie einer Tablette, einer Kapsel oder eines Beutels enthaltend einen ACE-Inhibitor oder ein pharmazeutisch unbedenkliches Salz davon als Wirkstoff und eine wirksame Menge eines anorganischen Siliciumdioxidpolymers wie Dimethicon als Stabilisator zur Inhibierung von Cyclisierung und/oder Hydrolyse und/oder Oxidation, bei dem man:
- eine homogene Mischung bildet, indem man die Gesamtmenge an Wirkstoff und/oder die Gesamtchargenmenge oder einen Teil davon eines fakultativen Verdünnungsmittels und/oder die Gesamtchargenmenge oder einen Teil davon eines fakultativen Füllstoffs mischt;
- die obige Mischung durch ein Sieb siebt und anschließend mit der Gesamtmenge der wirksamen Menge an Dimethicon mischt;
- die obige Mischung durch ein Sieb siebt;
- die gesiebte Mischung mit der Gesamtmenge aller anderen fakultativen Exzipienten versetzt und mischt, bis man eine einheitliche Mischung erhält, und
- die erhaltene Mischung als eine feste Dosierungsform formuliert, indem man sie entweder zu einer gewünschten Tablettenform komprimiert oder indem man Kapseln oder Beutel füllt.

15. Verfahren zur Herstellung einer festen Dosierungsform zur oralen oder sublingualen Verabreichung wie einer Tablette, einer Kapsel oder eines Beutels enthaltend einen ACE-Inhibitor oder ein pharmazeutisch unbedenkliches Salz davon als Wirkstoff und eine wirksame Menge eines anorganischen Siliciumdioxidpolymers wie Dimethicon als Stabilisator zur Inhibierung von Cyclisierung und/oder Hydrolyse und/oder Oxidation, bei dem man:
- eine erste Mischung der Gesamtmenge des Wirkstoffs mit einer wirksamen Menge eines anorganischen Siliciumdioxidpolymers wie Dimethicon als Stabilisator und/oder der Gesamtchargenmenge oder eines Teils davon eines fakultativen Verdünnungsmittels und/oder der Gesamtchargenmenge oder eines Teils davon eines fakultativen Füllstoffs bildet;
- gegebenenfalls einen zusätzlichen Wirkstoff mit der Gesamtmenge wenigstens eines fakultativen Exzipienten wie eines Bindemittels, eines Sprengmittels, eines Schmiermittels und/oder eines Gleitmittels mischt, bis man eine einheitliche Mischung erhält, und durch Zugabe eines wasserfreien Granuliermediums wie absolutem Ethanol, Aceton oder Mischungen davon feucht granuliert;
- das feuchte Material trocknet;
- das getrocknete Material zum Erzielen der gewünschten Granulatgröße siebt;
- das getrocknete Material zur ersten Mischung gibt und mischt und
- die erhaltene Mischung als eine feste Dosierungsform formuliert, indem man sie entweder zu einer gewünschten Tablettenform komprimiert oder indem man Kapseln oder Beutel füllt.

16. Verfahren nach einem der Ansprüche 13 bis 15, wobei es sich bei dem Wirkstoff um Fosinopril oder ein Salz davon handelt.

17. Verfahren nach einem der Ansprüche 13 bis 15, wobei es sich bei dem Wirkstoff um Trandolapril oder ein Salz davon handelt.

18. Verfahren nach Anspruch 13 oder 14, wobei die gesiebte Mischung weiterhin mit wenigstens einem zusätzlichen Wirkstoff ausgewählt aus der Gruppe der Diuretika wie Hydrochlorothiazid oder der Gruppe der Calciumkanalblocker wie Verapamil oder der Gruppe der Betablocker versetzt wird.

## Revendications

1. Composition pharmaceutique pour une administration par voie orale ou sublinguale comprenant un inhibiteur d'ECA ou un sel pharmaceutique acceptable de celui-ci, à titre de principe actif, et une quantité efficace de polymère de silice minéral, tel que la diméthicone, à titre de stabilisant pour inhiber la cyclisation et/ou l'hydrolyse et/ou l'oxydation.

2. Composition pharmaceutique selon la revendication 1, ledit polymère de silice minéral étant la diméthicone.

3. Composition pharmaceutique selon la revendication 2, qui comprend de 0,5 à 50 % en poids dudit inhibiteur d'ECA ou sel de celui-ci, et de 0,1 à 25 % en poids de ladite diméthicone.

4. Composition pharmaceutique selon la revendication 2, le rapport pondéral dudit inhibiteur d'ECA ou sel de celui-ci sur la diméthicone étant de préférence compris entre 500:1 et 1:50 et plus préférablement compris entre 200:7,5 et 7,5:80.

5. Composition pharmaceutique selon la revendication 1, qui comprend d'environ 0,5 % à 40 %, plus préférablement de 0,75 % à 25 % et le plus préférablement de 0,75 % à 20 % en poids dudit inhibiteur d'ECA ou sel de celui-ci.

6. Composition pharmaceutique selon la revendication 2, qui comprend d'environ 0,2 % à 20 %, plus préférablement de 0,5 % à 15 % et le plus préférablement de 0,75 % à 10 % en poids de diméthicone.

7. Composition pharmaceutique selon l'une quelconque des revendications précédentes, ledit inhibiteur d'ECA étant le fosinopril ou un sel de celui-ci.

8. Composition pharmaceutique selon l'une quelconque des revendications précédentes, ledit inhibiteur d'ECA étant le trandolapril ou un sel de celui-ci.

9. Composition pharmaceutique selon l'une quelconque des revendications précédentes, qui comprend en outre au moins un principe actif supplémentaire.

10. Composition pharmaceutique selon la revendication 9, ledit principe actif supplémentaire étant choisi dans le groupe des diurétiques tels que l'hydrochlorothiazide ou dans le groupe des agents bloquant les canaux calciques tels que le vérapamil ou dans le groupe des bêta-bloquants.

11. Composition pharmaceutique selon l'une quelconque des revendications précédentes, qui comprend en outre au moins un excipient éventuel choisi dans le groupe constitué de diluants, de liants, de délitants, de lubrifiants et d'agents de glissement.

12. Composition pharmaceutique selon l'une quelconque des revendications précédentes, ladite composition se présentant sous une forme galénique solide telle qu'un comprimé, une capsule ou un sachet comprenant un principe actif tel que le fosinopril ou le trandolapril ou des sels de ceux-ci.

13. Procédé de préparation d'une forme galénique solide pour une administration orale ou sublinguale, telle qu'un comprimé, une capsule ou un sachet, contenant un inhibiteur d'ECA ou un sel pharmaceutique acceptable de celui-ci à titre de principe actif et une quantité efficace de polymère de silice minéral, tel que la diméthicone, à titre de stabilisant pour inhiber la cyclisation et/ou l'hydrolyse et/ou l'oxydation, ledit procédé comprenant les étapes consistant à :
- former un mélange homogène en mélangeant la quantité totale dudit principe actif avec la quantité totale de ladite diméthicone ;
- tamiser le mélange ci-dessus à travers un tamis ;
- ajouter au mélange tamisé les quantités totales d'au moins un excipient éventuel tel qu'un liant, un diluant, un délitant, un lubrifiant et/ou un agent de glissement et mélanger jusqu'à homogénéité, et
- formuler le mélange résultant en une forme galénique solide soit en le comprimant en une forme de comprimé souhaitée soit en l'introduisant dans des capsules ou sachets.

14. Procédé de préparation d'une forme galénique solide pour une administration orale ou sublinguale, telle qu'un comprimé, une capsule ou un sachet, contenant un inhibiteur d'ECA ou un sel pharmaceutique acceptable de celui-ci à titre de principe actif et une quantité efficace de polymère de silice minéral, tel que la diméthicone, à titre de stabilisant pour inhiber la cyclisation et/ou l'hydrolyse et/ou l'oxydation, ledit procédé comprenant les étapes consistant à :
- former un mélange homogène en mélangeant la quantité totale dudit principe actif et/ou la quantité totale du lot ou une partie de celui-ci d'un diluant éventuel, et/ou la quantité totale du lot ou une partie de celui-ci d'une charge éventuelle ;
- cribler le mélange ci-dessus à travers un crible et le mélanger par la suite avec la quantité totale de la quantité efficace de diméthicone ;
- tamiser le mélange ci-dessus à travers un tamis ;
- ajouter au mélange tamisé la quantité totale de tout autre excipient éventuel et mélanger jusqu'à homogénéité, et
- formuler le mélange résultant en une forme galénique solide soit en le comprimant en une forme de comprimé souhaitée soit en l'introduisant dans des capsules ou sachets.

15. Procédé de préparation d'une forme galénique solide pour une administration orale ou sublinguale, telle qu'un comprimé, une capsule ou un sachet, contenant un inhibiteur d'ECA ou un sel pharmaceutique acceptable de celui-ci à titre de principe actif et une quantité efficace de polymère de silice minéral, tel que la diméthicone, à titre de stabilisant pour inhiber la cyclisation et/ou l'hydrolyse et/ou l'oxydation, ledit procédé comprenant les étapes consistant à :
- former un premier mélange de la quantité totale dudit principe actif avec une quantité efficace de polymère de silice minéral, tel que la diméthicone, à titre de stabilisant et/ou la quantité totale du lot ou une partie de celui-ci d'un diluant éventuel et/ou la quantité totale du lot ou une partie de celui-ci d'une charge éventuelle ;
- mélanger, éventuellement, un principe actif supplémentaire avec la quantité totale d'au moins un excipient éventuel tel qu'un liant, un délitant, un lubrifiant et/ou un agent de glissement jusqu'à homogénéité et réaliser une granulation à l'état humide en ajoutant un milieu de granulation anhydre tel que de l'éthanol absolu, de l'acétone ou des mélanges de ceux-ci ;
- sécher la masse humidifiée ;
- tamiser le mélange séché pour obtenir la taille de granule souhaitée ;
- ajouter la matière séchée au premier mélange et les mélanger, et
- formuler le mélange résultant en une forme galénique solide soit en le comprimant en une forme de comprimé souhaitée soit en l'introduisant dans des capsules ou sachets.

16. Procédé selon les revendications 13 à 15, ledit principe actif étant le fosinopril ou un sel de celui-ci.

17. Procédé selon les revendications 13 à 15, ledit principe actif étant le trandolapril ou un sel de celui-ci.

18. Procédé selon la revendication 13 ou 14, selon lequel on ajoute en outre, dans ledit mélange tamisé, au moins un principe actif supplémentaire choisi dans le groupe des diurétiques tels que l'hydrochlorothiazide ou dans le groupe des agents bloquant les canaux calciques tels que le vérapamil ou dans le groupe de bêta-bloquants.
